# EUROPEAN PATENT SPECIFICATION

(11) **EP 1 267 849 B1**
(45) Date of publication and mention of the grant of the patent: **16.02.2005**
(21) Application number: 01909964.7
(22) Date of filing: 28.02.2001
(51) Int. Cl.: A61K 31/00, A61P 25/30

(54) **RESTRICTING REINSTATEMENT OF DRUG USE**
EINDÄMMUNG DER WIEDERAUFNAHME VON DROGENMISSBRAUCH
SUBSTANCES DIMINUANT LA REAPPARITION DE L'USAGE DE DROGUES

(30) Priority: 28.02.2000 GB 0004708
(43) Date of publication of application: 02.01.2003
(73) Proprietor: BRITANNIA PHARMACEUTICALS LIMITED, Redhill, Surrey RH1 6YS (GB)
(72) Inventor: DAVIES, Keith, Redhill Surrey RH1 6YS (GB); LE, Dzung, Anh, Toronto, Ontario (CA)
(74) Representative: Blake, John Henry Francis
(86) International application number: PCT/GB2001/000847
(87) International publication number: WO 2001/064201

(56) References cited:
- WO-A-96/14071
- PT-A- 99 629
- US-A- 4 382 946
- US-A- 4 783 456
- US-A- 4 800 209
- US-A- 5 272 149
- US-A- 5 789 411
- VINING E ET AL: "CLINICAL UTILITY OF RAPID CLONIDINE-NALTREXONE DETOXIFICATION FOR OPIOID ABUSERS" BRITISH JOURNAL OF ADDICTION, CARFAX PUBLISHING, ABINGTON, GB, vol. 83, no. 5, 1 May 1988 (1988-05-01), pages 567-575, XP000578953 ISSN: 0952-0481
- BREWER C ET AL: "OPIOID WITHDRAWAL AND NALTREXONE INDUCTION IN 48-72 HOURS WITH MINIMAL DROP-OUT, USING A MODIFICATION OF THE NALTREXONE-CLONIDINE TECHNIQUE" BRITISH JOURNAL OF ADDICTION, CARFAX PUBLISHING, ABINGTON, GB, vol. 153, 1 September 1988 (1988-09-01), pages 340-343, XP000578954 ISSN: 0952-0481

## Description

This invention relates to pharmaceutical treatments for preventing or reducing relapse into addictive drug use under stress.

When a mammal has become addicted to an addictive material, the mammal may abstain from further use due to non-availability of the addictive material, due to physical or mental treatment, or simply by willpower. Under most circumstances the abstaining addict will relapse, and re-establish use of the addictive material, due to craving for the substance, or due to social or peer pressure.

In humans, experimental evidence shows that psychological stress can provoke craving for cocaine in cocaine addicts. In animal models of stress-induced relapse into addictive drug seeking, stress in the form of electric foot-shock has been shown to induce the relapse of rats addicted to heroin, cocaine, nicotine and alcohol. (This is sometimes referred to as "reinstatement" of drug use).

It has previously been proposed that clonidine and lofexidine can be used to alleviate withdrawal symptoms in patients being treated for addiction to opiates. For example the use of some ∝-2 adrenergic receptor agonists in treating the short term side effects of drug withdrawal is known from US 4 800 209. Euro. J. Neurosci., Vol. 12, pp 292-302, 2000, provides data indicating that clonidine is also effective in reducing foot-shock-induced relapse into heroin-seeking in heroin-addicted rats.

On the basis of further foot-shock data, the present invention proposes treatment regimes for ethanol and nicotine (i.e. non-opiates) addiction based on administration of ∝-2 adrenergic receptor agonists to prevent relapse under stress after extinction of desire for the substance.

For the treatment of addicts to ethanol or nicotine, existing therapies rely principally on abstinence programmes with peer support. After such programmes, relapse under stress can be alleviated in accordance with the present invention, by administration of ∝-2 adrenergic receptor agonists.

Therefore the present inventors have devised a method of preventing or reducing stress-induced relapse which comprises treating a mammal addicted to ethanol or nicotine after extinction of desire for the substance by administering an effective amount of an ∝-2 adrenergic receptor agonist.

In this method it is expected that the addict will be refraining from use of the addictive material during the treatment and so the addict will be administered solely the ∝-2 adrenergic receptor agonist.

According to the invention there is provided the use of an ∝-2 adrenergic receptor agonist in the manufacture of a medicament for use in the treatment regimes indicated above.

The use of the α-2 adrenergic receptor agonist may be combined with a programme of treatment with an agonist for the addictive substance, or a compound that produces an unpleasant effect, such as nausea, if there is illicit use of the addictive substance.

The use of an α-2 adrenergic receptor agonist in accordance with this invention has also been found to be effective against relapse (reinstatement) of multiple drug abuse.

In this invention, it may still be necessary initially to treat acute withdrawal symptoms with, for example, clonidine or lofexidine, or with a medicament other than an alpha 2 adrenergic receptor agonist, such as the nonapeptide described in US 4 1496 545.

Whereas treatment of withdrawal symptoms with ∝-2 adrenergic receptor agonists requires administration of these compounds for periods of a few days, treatment to reduce the risk of relapse in accordance with the present invention will typically require administration of the compounds for several months.

Examples of suitable alpha-2 adrenergic receptor agonists include guanabenz, lofexidine and clonidine. Lofexidine is preferred.

Compounds such as agonists or substitutes for the addictive substance are administered in accordance with their normal regimes.

Those skilled in the art will be able to devise suitable means for administering the alpha-2 adrenergic receptor agonist, which may be salt form or pro-drug form. The precise method adopted will depend on the material selected examples of possible administration routes may be oral, nasal, transdermal, ip and iv.

The active compound will normally be employed in the form of a pharmaceutical composition in association with a pharmaceutical carrier, diluent and/or excipient, although the exact form of the composition will naturally depend on the mode of administration. Administration by transdermal patches is psychologically advantageous in providing addicts with a visible or touchable reminder that medication is being provided. Such patches are prepared in conventional manner typically as a gel formulation containing a permeation enhancer.

Compositions may be prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administerable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art. Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability; the composition can be frozen after filling into the vial and the water removed under vacuum. Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

Compositions used in this invention may be formulated by conventional means for controlled or delayed release.

More specifically, clonidine and lofexidine may suitably be administered in formulations previously used in the existing regimes to treat withdrawal symptoms.

As is common practice, the compositions will usually be accompanied by written or printed directions for use in the medical treatment concerned. The composition is preferably presented as a unit dose composition.

Those skilled in the art will be able to determine by routine experiment suitable dosage levels which will depend on the active material and administration route. It is anticipated that the dosages for use against relapse will be less than those used in treating withdrawal symptoms, for example, in the region of 0.6 to 3.2 mgs. in the case of lofexidine.

Lofexidine, clonidine and guanabenz are commercially available compounds. No toxic effects are anticipated in the use of these compounds in accordance with this invention.

While the applicants do not wish to be bound by this theory, it is thought that the intervention between the brain noradrenergic (NE) system and the alpha-2 adrenergic receptor agonists gives rise to the beneficial effects of the invention.

Non-limiting examples of the invention and control experiments will now be described by reference to the Figures of the accompanying drawings of which:

### Figure 1.

### Lofexidine - effect on alcohol self-administration

Effects of pre-treatment with saline, 0.05 or 0.1 mg/kg of lofexidine on alcohol self-administration as shown by amount of alcohol self-administered in g/kg during a 1-hour operant session (left panel) or number of alcohol reinforcements obtained (right panel). Values presented are averages of two consecutive sessions. N=9-10 animals per group. *P<0.05 compared to saline control.

### Figure 2.

### Lofexidine ― effect on extinction of alcohol self-administration

Effects of pre-treatment with saline and lofexidine on extinction of alcohol self-aministration as measured by number of responses on the active lever across eight operant sessions. N=9-10 animals per group. *P<0.05 compared to saline treatment.

### Figure 3.

### Lofexidine - effect on reinstatement (relapse) of alcohol self-administration

Effects of pre-treatment with saline, 0.05, 0.1 and 0.2 mg/kg of lofexidine on reinstatement of alcohol-seeking as measured by responses on the active lever induced by exposure to 10-minutes of intermittent footshock stress. N=10-12 animals per group. # P<0.05 compared to baseline or drug only conditions. *P<0.5 compared to saline shock condition.

### Figure 4.

### Lofexidine - effect on reinstatement (relapse) of alcohol self-administration

Effects of pre-treatment with saline, 0.05, 0.1 and 0.2 mg/kg of lofexidine on responses on the inactive lever induced by exposure to 10 minutes of intermittent footshock stress. N=10-12 rats per group.

### ACTIVITY AGAINST REINSTATEMENT OF ETHANOL USE

### MATERIALS AND METHODS

***Animals:*** Male Wistar rats (Charles River, Montreal; weighing about 150-200 g. at the start of the experiments) were individually housed. Food and water were freely available in the home cages for 23 h/day. Food was not available during the 30 to 60 min daily session (see below). The temperature was maintained at 21± 1°C and the lights were on from 7 a.m. to 7 p.m. Rats were initially trained to consume alcohol in a limited access procedure (Linseman, 1987). The rats were given access to an alcohol solution and water in modified Richter tubes for 30 min/day in drinking cages. Alcohol solutions were provided in escalating concentrations: 3% w/v for the first 5 days 6% for the next 8 days and 12% for the last 10-12 days. Rats that consumed less than 0.4 g/kg per session during the last 10-12 days were excluded. All procedures were done in accordance with the guidelines of the Canadian Council on Animals Care and the 'principle of laboratories animal care' (NIH publication No. 85-23, revised 1985), and were approved by the local Animal Care Committee.

***Apparatus:*** The alcohol self-administration system was built locally. Each operant chamber was equipped with two levers, symmetrically centered on the side panel. Responding on one lever (the active lever) activated the infusion pump (Razel Sci. Stamford, Conn., USA). Presses on the other lever (the inactive lever) were recorded, but did not activate the pump. Activation of the pump resulted in the delivery of 0.19 ml of 12% w/v alcohol solution to a liquid drop receptacle located between the two levers over a period of 5 s. During the infusion, a stimulus light above the active lever was turned on for 6 s. Lever presses during this 'time out' period were counted, but did not lead to further infusions. The grid floor of the operant cambers was connected to electric shock generators (Med Associates, Georgia, Vt., USA).

***Training and extinction of alcohol self-administration:*** Operant self-administration of alcohol was initiated on a fixed ration-1 schedule of reinforcement (FR-1, each lever press is reinforced) for 60 min per day for 5-7 days per week. Responding on the FR-1 schedule was maintained for 10-14 sessions. The requirement for alcohol delivery was then increased to FR-2 for five sessions. Subsequently, the schedule requirement was increased to an FR-3 schedule for 8-12 days until the rats obtained three days of stable drug taking (variability of less than 20% of the mean).
During extinction sessions, presses on the previously active lever had no programmable consequences. Test for reinstatement (relapse) commenced after five to eight extinction sessions, when the rats reached the extinction criterion of fewer than 12 presses on the active lever per 60 min.

***Test for Reinstatement:*** Tests were conducted under extinction conditions. Intermittent footshock (0.8 mA; 0.5 s ON; a mean OFF period of 40 s; a range of 10-70 s) was administered for a period of 10 min just prior to the test sessions. Footshock was delivered through a scrambler to the stainless steel grid floor of the operant chambers.

***Drug:*** Lofexidine HCL was provided by Britannia Pharmaceuticals Ltd. The drug was dissolved in saline and injected IP in a volume of 1 ml per kg. Lofexidine, pre-treatment was given one hour prior to the start of the operant sessions.

### Experiment 1:

### Effects of Lofexidine on alcohol self-administration and extinction

This experiment was designed to examine the effect of different doses of lofexidine on alcohol self-administration and extinction of alcohol self-administration behaviour.
Three groups of rats trained to attain a stable and high level of alcohol self-administration were used. One group of rats received saline treatment while rats from the other two groups received 0.1 and 0.05 mg/kg of lofexidine treatment one hour prior to the operant session. The effect of lofexidine on alcohol self-administration was examined on two consecutive sessions. Animals received IP injection of saline 1 hour prior to the operant session for three sessions prior to testing to habituate the animal with the injection procedure.

All rats then received daily injection with saline 60 min prior to the operant sessions for 4 days. To examine the effect of lofexidine on extinction of ethanol self-administration behaviour, rats received daily treatment with lofexidine (0.05 or 0.1 mg/kg) or saline one hour prior to their daily operant session. During this phase, responding on the active lever did not result in any alcohol delivery.

### Experiment 2:

### Effect of Lofexidine on footshock reinstatement of alcohol seeking

Three groups of rats (n=10 each) which attained stable alcohol self-administration were used for this study. When the animals achieved extinction criterion, the effect of pre-treatment with lofexidine on footshock stress-induced reinstatement (relapse) of alcohol seeking was determined as described above. Rats from group 1 received saline while rats from group 2 and 3 received treatment with 0.05 or 0.1 mg/kg of lofexidine 1 hour prior to exposure to footshock stress.

### Data Analysis

Analysis of variance with or without repeated measures were employed to analyse the data. Where appropriate, post-hoc Newman Keuls' t tests were used. In the first experiment, the average amounts of alcohol self-administered over a period of two days were used to quantify the effects of lofexidine treatment on alcohol self-administration. Such data are expressed in terms of g/kg of alcohol consumed by the animals during the 1-hour session. This data is derived from multiplying the amount of alcohol delivered per reinforcement (23.8 mg of alcohol) with the number of reinforcement obtained and divided by the animal body weight.

The number of responses on the active lever are used to assess the rate of extinction and the effects of lofexidine on extinction rate. This parameter is also used to assess the effects of footshock stress on reinstatement (relapse) of alcohol seeking.

### RESULTS

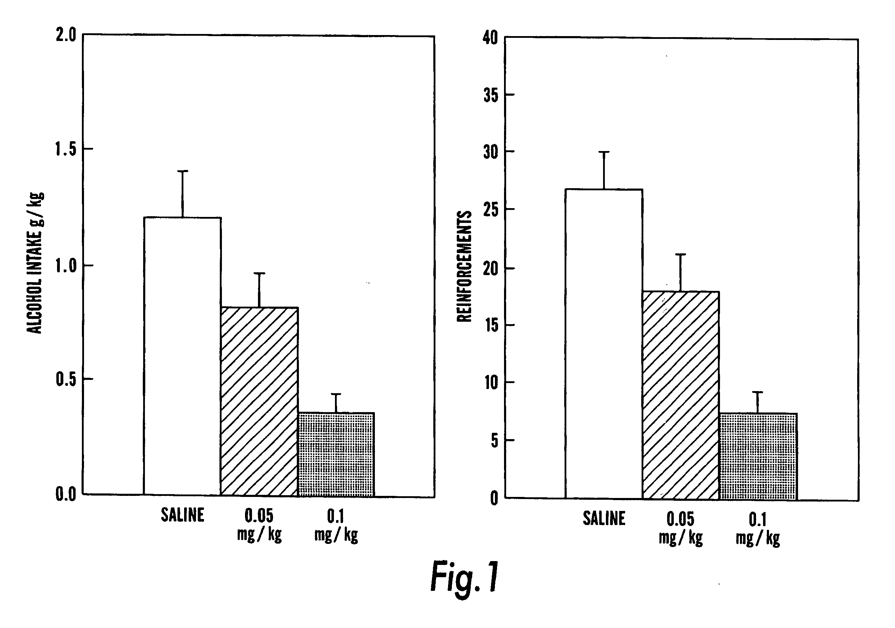

**Experiment 1:** Figure 1 shows the average alcohol self-administration in various experimental groups over two days of lofexidine or saline treatment. As seen from this figure, rats self-administered an average of about 1.4 g/kg during their daily 1-hour session. Analysis of variance reveals a significant treatment effect (F=9.05, df=2,28) which indicates that lofexidine treatment affects alcohol self-administration. Post-hoc Newman-Keuls tests show that alcohol self-administration in rats treated with 0.1 mg/kg of lofexidine was significantly lower (p<0.05) than those of the saline or 0.05 mg/kg lofexidine treated group. Similar tests show no significant differences in the amount of alcohol self-administration between the saline and 0.05 mg/kg lofexidine group. These results indicate that pre-treatment with 0.1 mg/kg but not 0.05 mg/kg of lofexidine significantly reduced alcohol self-administration.

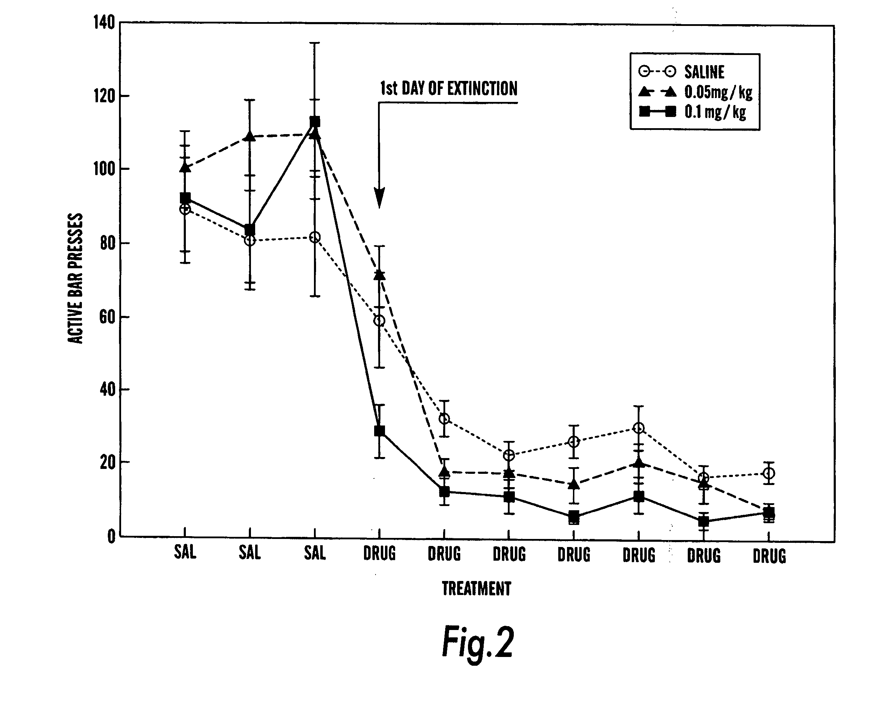

The effect of lofexidine pre-treatment on the rate of extinction of ethanol self-administration behaviour as measured by the number of responses on the active lever is shown in Figure 2. An overall analysis of variance comparing the responses on the active lever of various groups on the last session prior to extinction and the 7 extinction sessions reveals a significant Group x Day interaction (F=2.35, df=14, 184, p<0.01) which indicates that the rates of change in the response on the active lever across different sessions varied with the treatment. Since the reduction in responses on the active lever was at maximal by the second session of extinction, an analysis of variance was carried out to examine the responses on the last session prior to extinction and the first two extinction sessions. Post-hoc Newman-Keuls tests show no significant differences in the number of responses on the active lever between the 0.05 mg/kg and 0.1 mg/kg lofexidine treated group on the last session prior to extinction. The responding on both lofexidine treated groups on this session, however, was significantly higher than the saline treated group. On the first day of extinction, responding on the active lever in the 0.1 mg/kg treated group was significantly lower than that of the 0.5 mg/kg and the saline treated group. There were no significant differences in responding on the active lever among the three groups by the second day of extinction. These results indicate that treatment with 0.1 mg/kg of lofexidine might facilitate extinction of alcohol self-administration behaviour.

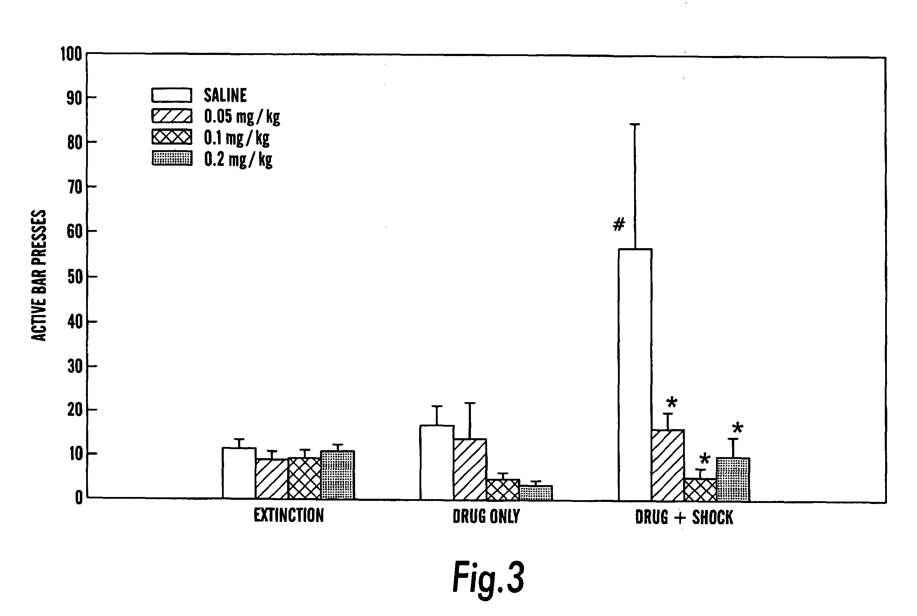

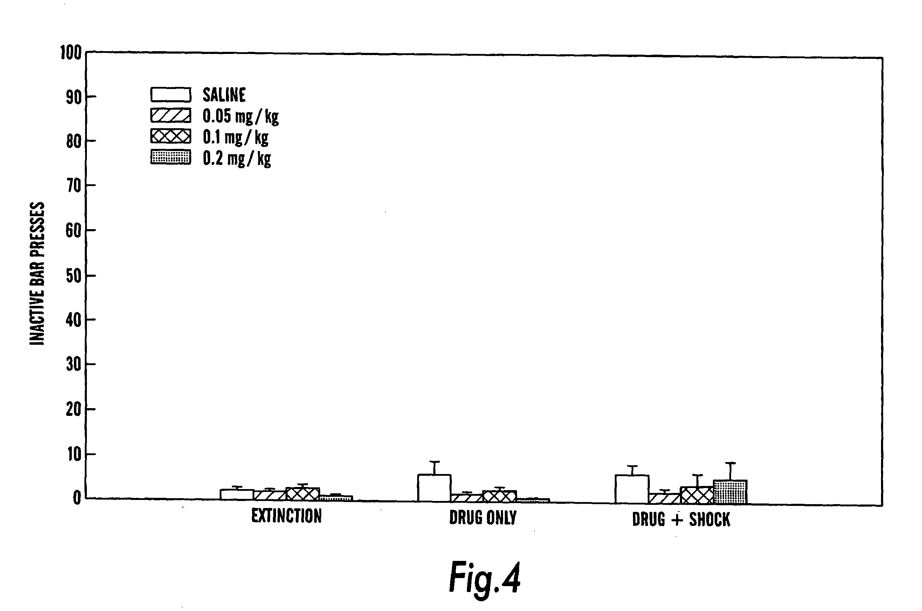

**Experiment 2:** The effects ofpre-treatment with saline and various doses of lofexidine on footshock stress induced-responding on the active and inactive levers are shown in Figure 3 and Figure 4 respectively. Exposure to footshock stress potently reinstates alcohol seeking in the saline treated group as indicated by an increase in responding on the active lever. Pre-treatment with 0.05, 0.1 or 0.2 mg/kg of lofexidine significantly suppressed reinstatement (relapse) of alcohol seeking. An analysis of variance comparing the responses of various groups on the active bar showed a significant effect of treatment (F=3.53 df=3.38, p<0.02). Post-hoc tests show that the responses on the active lever of all the lofexidine treated groups following exposure to footshock were not different from one another. They are, however, all significantly below that of the saline treated group (p<0.05). These results indicated that all doses of lofexidine suppressed reinstatement (relapse) of alcohol seeking.

Such exposure to footshock stress did not increase responding on the active lever in saline and various lofexidine treated groups as compared to their baseline responses (F=2.3, df=3.38, p=0.09). These results indicate that the increase in response to the active lever induced by footshock stress was not due to a generalized increase in activity.

### DISCUSSION

The results of these studies confirm and extend previous observations that with prior exposure to alcohol drinking in a limited access paradigm rats will readily self-administer alcohol in an operant paradigm. Rats self-administer an average of 1.2-1.3 g/kg during 1-hour operant session. The amount of alcohol self-administered exceeded the metabolic capacity and produced significant blood alcohol levels (average of 55 mg%, with a range). The present study demonstrated that treatment with the alpha-adrenergic receptor agonist, lofexidine can reduce alcohol self-administration; tended to facilitate extinction of alcohol self-administration behaviour and also attenuated stress-induced relapse to alcohol seeking behaviour.

Pre-treatment with lofexidine at a dose of 0.1 mg/kg significantly suppresses alcohol self-administration as measured over two consecutive days of treatment. A reduction in alcohol self-administration was also observed with the lower dose of lofexidine. Such effects, however, did not reach statistical significance. Our findings are consistent with reports of a reduction in alcohol consumption by pre-treatment with various α-2-adrenergic receptor agonists such as clonidine, guanfacine and tiamedine. It is possible that the decrease in central noradrenergic activity induced by lofexidine or clonidine might account for its action in reducing alcohol self-administration. Studies which examined the effects of lesioning central noradrenergic systems on alcohol consumption, have produced some conflicting results. For example, depletion of brain noradrenaline has been shown to increase or decrease alcohol consumption in rats. It has also been suggested that an intact noradrenergic system is necessary to maintain alcohol consumption. The present findings with lofexidine, a drug that produces minimal hypotensive effect, on operant self-administration of alcohol suggest that the brain noradrenergic systems might play a role in the rewarding effects of alcohol.

Although, all rats received a similar pre-treatment with saline on the last day of alcohol self-administration, the responding on the active lever of the designated saline treated group was significantly lower than those of animals designated to receive 0.05 and 0.1 mg/kg treatment. We do not have any explanation for such a difference. However, responses on the active lever were essentially the same in both the 0.05 and 0.1 mg/kg groups. There was a significant decrease in the number of bar presses on the active lever in rats treated with 0.1 mg/kg of lofexidine as compared to the saline control or the 0.05 mg/kg treated group on day 1 of extinction. This suggests that lofexidine at a dose of 0.1 mg/kg facilitates extinction of alcohol self-administration behaviour. Such facilitation of the extinction of alcohol self-administration behaviour suggests that lofexidine reduces alcohol seeking or reduces the desire for alcohol. This interpretation would be consistent with the observation of a reduction in alcohol self-administration mentioned above. Alternatively, since the brain noradrenergic systems have been implicated in learning, one could suggest that the facilitation of extinction of alcohol self-administration might be due to an effect of lofexidine on learning i.e. extinction in a learned behaviour.
However, it is unlikely that such learning factor plays any significant role on the observed effect of lofexidine as the maximal extinction effect was observed on the first day of lofexidine treatment.

In agreement with our previous findings, exposure to intermittent footshock stress potently reinstates alcohol seeking after alcohol self-administration behaviour has been extinguished. Pre-treatment with lofexidine in doses ranging from 0.05 to 0.2 mg/kg completely suppressed reinstatement of alcohol seeking. Thus, it appears that stress-induced reinstatement (relapse) of alcohol seeking is more sensitive to lofexidine than alcohol self-administration and extinction of such self-administration.

A possible factor that might confound the interpretation of the present work is that lofexidine might reduce alcohol self-administration, facilitate its extinction and block stress-induced reinstatement (relapse) of alcohol seeking by impairing motor performance. It is unlikely that lofexidine affects various alcohol related behaviours by producing motor deficits. It is shown above that doses of lofexidine ranging from 0.08 mg to 0.2 mg/kg did not affect responding to 30% sucrose solution.

Lofexidine, in doses employed in the present study have been shown to block stress induced NE release in the pre-frontal cortex as well as the amygdala. It is tempting to suggest that the observed effects of lofexidine on various alcohol related behaviours examined in the present study are due to its action on α-adrenergic receptors. However, we cannot rule out the possibility that lofexidine might also mediate its action through the imidazoline-type 1 (I2) receptors as it also has high affinity for these receptors. Independent of its mechanism of action, the results of the present study point out that lofexidine might have important therapeutic applications for treatment of alcohol abuse. Its suppression of alcohol self-administration as well as its attenuation of stress-induced alcohol seeking observed in the present study strongly suggest further investigation for its use in alcohol relapse prevention.

### SUMMARY

It seems unlikely that footshock-induced relapse (reinstatement of alcohol seeking) is mediated by analgesic effects of the alcohol at the doses used. Observations made during the footshock sessions revealed that both vehicle- and drug-pretreated animals reacted similarly to the footshocks throughout the shock period. These observations are consistent with the finding that clonidine, within a similar dose range to the one used in the present study, does not alter threshold sensitivity to footshock.

In addition, the findings presented here indicate the utility of α-2 adrenergic receptor agonists, in particular lofexidine and clonidine, in treatment programs for the prevention of relapse to nicotine and alcohol use. The current use of these drugs in the short-term treatment of acute opioid withdrawal, though useful, does not prevent relapse to drug use following its termination. According to the invention a more prolonged period of treatment is efficacious.

Thus the invention provides a rationale for the use of *alpha*-2 adrenergic receptor agonists in treatment programs for the prevention of relapse to ethanol use. Currently, these drugs are being used with some success in the short-term treatment of opioid withdrawal. The present findings surprisingly indicate utility in the treatment of ethanol users if given for a more prolonged period. Also the present experiments surprisingly demonstrate a similar efficacy of clonidine and lofexidine in preventing footshock-induced relapse. This finding is of potential clinical significance in that lofexidine has been reported in humans to be associated with fewer adverse side effects than clonidine, in particular fewer hypotensive effects.

## Claims

1. Use of an alpha-2 adrenergic receptor agonist, a physiologically acceptable salt thereof or a pro-drug thereof, in the manufacture of a medicament for preventing or reducing the stress-related relapse into nicotine or ethanol-seeking.

2. A use as claimed in claim 1 in which the alpha-2 adrenergic receptor agonist is lofexidine, clonidine or guanabenz.

3. A use as claimed in claim 1 in which lofexidine or a physiologically acceptable salt or pro-drug thereof is used in the manufacture of a medicament.

4. A use as claimed in claim 2 or 3 in which lofexidine is provided as lofexidine hydrochloride.

5. Use of lofexidine, a physiologically acceptable salt thereof or a pro-drug thereof in the manufacture of a medicament for preventing or reducing the stress-induced relapse into ethanol-seeking in a mammal addicted to ethanol but in which there is an extinction of desire of the use thereof.

## Patentansprüche

1. Verwendung eines Alpha-2 adrenergen Rezeptor-Agonisten, eines davon abgeleiteten Salzes oder einer davon abgeleiteten Medikamentenvorstufe zur Herstellung eines Medikaments zur Verhinderung oder zur Verringerung der Wahrscheinlichkeit eines Stress bedingten Rückfalls in eine Nikotin- oder Alkoholsucht.

2. Verwendung nach Anspruch 1 bei welcher der Alpha-2 adrenerge Rezeptor-Agonist Lofexidin, Clonidin oder Guanabenz ist.

3. Verwendung nach Anspruch 1 bei welcher Lofexidin oder ein hiervon abgeleitetes physiologisch akzeptables Salz oder eine hiervon abgeleitete Medikamentenvorstufe zur Herstellung eines Medikaments verwendet wird.

4. Verwendung nach einem der Ansprüche 2 oder 3 bei welcher Lofexidin in Form von Lofexidin Chlorhydrat angewandt wird.

5. Verwendung von Lofexidin oder eines hiervon abgeleiteten physiologisch akzeptablen Salzes oder einer hiervon abgeleiteten Medikamentenvorstufe, um bei Säugern einen Stress bedingten Rückfall in eine Alkoholsucht für einen trockenen Alkoholiker zu verhindern oder die Wahrscheinlichkeit dafür zu verringern.

## Revendications

1. Utilisation d'un agoniste des récepteurs alpha-2 adrénergiques, d'un sel acceptable sur le plan physiologique de celui-ci ou d'un promédicament de celui-ci, dans la fabrication d'un médicament destiné à prévenir ou à réduire la récidive, liée au stress, du besoin en nicotine ou en éthanol.

2. Utilisation selon la revendication 1, dans laquelle l'agoniste des récepteurs alpha-2 adrénergiques est la lofexidine, la clonidine ou le guanabenz.

3. Utilisation selon la revendication 1, dans laquelle la lofexidine, un sel acceptable sur le plan physiologique ou un promédicament de celle-ci est utilisé(e) dans la fabrication d'un médicament.

4. Utilisation selon la revendication 2 ou 3, dans laquelle la lofexidine est proposée sous forme de chlorhydrate de lofexidine.

5. Utilisation de la lofexidine, d'un sel acceptable sur le plan physiologique de celle-ci ou d'un promédicament de celle-ci dans la fabrication d'un médicament destiné à prévenir ou à réduire la récidive, induite par le stress, du besoin en éthanol chez un mammifère dépendant à l'éthanol mais chez lequel il y a une extinction du désir de recourir à celui-ci.
